# EUROPEAN PATENT APPLICATION

(11) **EP 1 101 824 A2**
(43) Date of publication of application: **23.05.2001**
(21) Application number: 00308509.9
(22) Date of filing: 28.09.2000
(51) Int. Cl.: C12Q 1/68

(54) **Detection of sequence variation of nucleic acid by shifted termination analysis**

(30) Priority: 22.11.1999 US 166898 P; 17.07.2000 US 618129
(71) Applicant: Wang, Xiao Bing, Lutherville, Maryland 21093 (US); Morisawa, Shinkatsu, Osaka-shi, Osaka (JP)
(72) Inventor: Wang, Xiao Bing, Lutherville, Maryland 21093 (US)
(74) Representative: Morton, Colin David

(57) **Abstract**

The invention relates to a method for detecting any mutation at a predetermined site occurring in a known nucleic acid sequence. The method uses primer extension analysis to detect the mutation. Unlabeled terminator is supplied along with labeled non-terminator in the primer extension reaction to detect whether the first nucleic acid base on the template strand that is directly opposite the nucleic acid base immediately 3' to a primer is a mutant. In the primer extension reaction, the terminator is complementary to the wild-type base on the template strand that is directly opposite the nucleic acid base immediately 3' to the primer. Non-terminators are the other nucleotides and are labeled. When the terminator is incorporated into the primer extension strand, primer extension reaction terminates. Incorporation of a labeled non-terminator in the primer extension strand indicates that a mutation has occurred at the predetermined nucleic acid base site.

### Annex to the application documents - subsquently filed sequences listing

SEQUENCE LISTING

<110> WANG, Xiao B.
      MORISAWA, Shinkatsu

<120> Detection of Sequence Variation of Nucleic Acid by
      Shifted Termination Analysis

<130> MJL/C617'1/W

<140> 00308509.9
<141> 2000-09-28

<160> 20

<170> PatentIn Ver. 2.1

<210> 1
<211> 30
<212> DNA
<213> Homo sapiens

<400> 1
cctggacaac catgccacca agcagaagta                                   30


<210> 2
<211> 30
<212> DNA
<213> Artificial Sequence

<220>
<223> Description of Artificial Sequence:synthetic
      oligonucleotide

<400> 2
cctggataac catgccacca agcagaagta                                   30


<210> 3
<211> 32
<212> DNA
<213> Artificial Sequence

<220>
<223> Description of Artificial Sequence:synthetic
      oligonucleotide

<400> 3
cctggatgta accatgccac caagcagaag ta                                32

<210> 4
<211> 28
<212> DNA
<213> Artificial Sequence

<220>
<223> Description of Artificial Sequence:synthetic
      oligonucleotide

<400> 4
cctggaacca tgccaccaag cagaagta                                     28

<210> 5
<211> 20
<212> DNA
<213> Artificial Sequence

<220>
<223> Description of Artificial Sequence:synthetic
      oligonucleotide

<400> 5
ttctgcttgg tggcatggtt                                              20

<210> 6
<211> 20
<212> DNA
<213> Homo sapiens

<400> 6
tccacctgaa cactatgttc                                              20

<210> 7
<211> 35
<212> DNA
<213> Artificial Sequence

<220>
<223> Description of Artificial Sequence:synthetic
      oligonucleotide

<400> 7
aggtggtgga ggtgttttac ttctgcttgg cggca                                35

<210> 8
<211> 35
<212> DNA
<213> Artificial Sequence

<220>
<223> Description of Artificial Sequence:synthetic
      oligonucleotide

<400> 8
aggtggtgga ggtgttttac ttcagcttgg cggca                                35

<210> 9
<211> 37
<212> DNA
<213> Artificial Sequence

<220>
<223> Description of Artificial Sequence:synthetic
      oligonucleotide

<400> 9
aggtggtgga ggtgttttac ttcgcagctt ggcggca                              37

<210> 10
<211> 34
<212> DNA
<213> Artificial Sequence

<220>
<223> Description of Artificial Sequence:synthetic
      oligonucleotide

<400> 10
aggtggtgga ggtgttttac ttcggcggca tggt                                 34

<210> 11
<211> 23
<212> DNA
<213> Artificial Sequence

<220>
<223> Description of Artificial Sequence:synthetic
      oligonucleotide

<400> 11
aggtggtgga ggtgttttac ttc                                              23


<210> 12
<211> 15
<212> DNA
<213> Artificial Sequence

<220>
<223> Description of Artificial Sequence:synthetic
      oligonucleotide

<400> 12
agtcnngcat gctga                                                       15


<210> 13
<211> 10
<212> DNA
<213> Artificial Sequence

<220>
<223> Description of Artificial Sequence:synthetic
      oligonucleotide

<400> 13
tcagcatgcn                                                             10


<210> 14
<211> 19
<212> DNA
<213> Artificial Sequence

<220>
<223> Description of Artificial Sequence:synthetic
      oligonucleotide

<400> 14
agtcnnnnnn gcatgctga                                                   19


<210> 15

<211> 11
<212> DNA
<213> Artificial Sequence

<220>
<223> Description of Artificial Sequence:synthetic
      oligonucleotide

<400> 15
tcagcatgcn n                                                          11


<210> 16
<211> 15
<212> DNA
<213> Artificial Sequence

<220>
<223> Description of Artificial Sequence:synthetic
      oligonucleotide

<400> 16
tcagcatgcn nnnnn                                                      15


<210> 17
<211> 18
<212> DNA
<213> Artificial Sequence

<220>
<223> Description of Artificial Sequence:synthetic
      oligonucleotide

<400> 17
agtcnnnnng catgctga                                                   18


<210> 18
<211> 14
<212> DNA
<213> Artificial Sequence

<220>
<223> Description of Artificial Sequence:synthetic
      oligonucleotide

<400> 18

tcagcatgcn nnnn 14

<210> 19
<211> 18
<212> DNA
<213> Artificial Sequence

<220>
<223> Description of Artificial Sequence:synthetic
      oligonucleotide

<400> 19
nnnnnnnnng catgctga 18

<210> 20
<211> 17
<212> DNA
<213> Artificial Sequence

<220>
<223> Description of Artificial Sequence:synthetic
      oligonucleotide

<400> 20
nnnnnnnngc atgctga 17

## Description

### BACKGROUND OF THE INVENTION

This invention relates to the field of nucleic acid sequence detection. The invention relates to a method of detecting any type of mutation at a predetermined nucleic acid base site of interest. The present invention is directed to a method called shifted termination analysis, also known as specific termination assay, or shifted terminator alignment which can all be abbreviated as STA.

Practical applications for the inventive method includes genetic disease diagnoses, infectious disease diagnoses, forensic techniques, paternity determinations, and genome mapping, wherein the site of the mutation to be detected is known.

In the past decade, genes implicated in inherited susceptibility to form cancer have been identified and many cancer-related mutations were characterized. Diagnostic tests for these mutations can provide a more accurate estimate of an individual's risk of developing cancer. Early diagnosis of a cancer related mutation is one of the goals of the invention.

There are four major types of gene mutations. The first is point mutations caused by a single nucleotide substitution in a normal DNA sequence. In most cases, this mutation causes a frame shift in the coding strand which results in termination of normal protein synthesis. A point mutation in APC gene as found in family adenopolyposis (FAP) patients is a typical example (Kinzler et al., *Science* 253, 661-665 (1991); Joslyn et al., *Cell* 66, 601-613 (1991); Nishisho et al., *Science* 253, 665-669 (1991)). The second is insertion mutation in which a single or multiple nucleotides are inserted into a normal DNA sequence. The third is deletion mutation, wherein a single nucleotide or multiple nucleotides are deleted from a normal DNA sequence. Both insertion and deletion types of mutations can cause severe changes such as frame shift, early termination of protein synthesis, and addition or deletion of one or multiple amino acids. The fourth is gene translocation, which occurs when a fragment of a gene is incorporated into another gene. The Philadelphia chromosome seen in chronic myeloid leukemia patients is an example of this phenomenon (Konopka et al., *Cell* 37 1035 (1984)). Changes in protein structure cause a series of disorders in a cell that can lead to the onset of cancer.

Detection of one mutated DNA among thousands of normal DNA is difficult. Chemical or enzymatic DNA sequencing method to directly read the DNA sequence of an isolated species, has been used as the most thorough method in research laboratory in analyzing and identifying gene mutations. However, clinical application of such sequencing method is impractical as it is limited by the level of professional skill that is required to perform the assays, its labor intensiveness, high cost associated with procurement of the apparati and reagents in carrying out the sequencing reactions, as well as the long duration required to complete the project. Finally, another disadvantage of the sequencing method is that the procedure requires a large amount of DNA template, which is difficult to obtain from a 10 ml blood specimen that is usually collected from a patient.

Examples of conventional mutation detection techniques include: restriction fragment length polymorphism (RFLP) (Botstein et al., *Am. J. Hum. Genet.,* 32, 314-331 (1980), and White et al., *Scientific American,* 258:40-48 (1988)); single-strand conformational polymorphism (SSCP) (Howell et al., *Am. J. Hum. Genet.,* 55, 203-206 (1994)); allele-specific oligonucleotide hybridization. (Studencki et al., *Am. J. Hum. Genet.,* 37, 42-51 (1985), and Saiki et al., *Nature,* 324, 163-166); oligonucleotide-ligation assay (Landgrun et al., *Science,* 241, 1077-1080 (1990)); and allele-specific PCR (ASPCR) (Wu et al., *Proc. Natl. Acad.Sci.,* 86, 2757-2760 (1989), and Okayama et al., *J. Lab. Clin. Med.* 114, 105-113 (1989)).

Some of these techniques are suitable for detecting only point mutations. Some of the other techniques can be used to detect only insertions or deletions that may for example, destroy or build up restriction enzyme cleavage sites, but are not suitable for detecting single base mutations. For example, point mutations that do not affect the enzyme cleavage site are missed by such methods as RFLP. Other techniques require optimization of a special probe hybridization condition. In addition, all of the above mentioned techniques require special laboratory equipment such as gel electrophoresis apparatus and hybridization equipment, time and labor.

Some primer extension based methods for detecting mutations are also known (Mohan et al., *Proc. Natl. Acad. Sci. USA,* 88, 1143-1147 (1991), Prezant et al., *Hum. Mutation* 1, 159-164 (1992), Fahy et al., *Nucleic Acid Research,* 25, 3102-3109 (1997), and U. S. Patent Nos. 5,846,710 (1998) and 5,888,819 (1998)). Such methods include: primer extension with a thionucleotide; primer extension from oligonucleotide primer flanking the mutated nucleotide with labeled nucleotide complementary to the mutated nucleotide base; and primer extension with labeled dideoxynucleotide terminator complementary to the mutant base.

These primer extension based mutation detection methods are fast, facile to perform, and can be potentially applied to clinical use. However, there are at least two weaknesses with these methods. First, all of these techniques are based on incorporating only one labeled-nucleotide in the primer extension strand. Incorporating only one type of labeled-nucleotide chosen from A, C, G, T or U , or labeled-dideoxynucleotide permits the detection of only the specific point mutation that is specific to the nucleotide base that is complementary to the labeled nucleotide that is used in the assay.

Hypothetically, when a different type or nature of mutation occurs at the same position, for example, if A is changed to C or GT, or TCT, with innumerable other permutations, these known methods require that at least three separate tests be conducted with labeled G, C, A. Alternatively, one test assay can be carried out that uses differentially labeled nucleotide combined with gel analysis and special marker detection system to detect the G, C, A mutants separately. However, carrying out three separate tests requires more than three times the blood sample that is generally obtained from patients. Such a high volume of blood sample or complicated gel analysis procedure not only increases the cost of the test, and the time and labor involved, but more importantly, the probability of error is increased because of chances of mislabeling of tubes and the numerous steps that are required to carry out these assays. Therefore, these primer extension based methods are not convenient or suitable for screening a large sample number or for carrying out routine tests at a clinic.

Second, the sensitivity of these primer extension based assays need improvement. Because the primer extended strand obtained in these tests carries only one labeled nucleotide or labeled dideoxynucleotide, the signals generated are varied and their strength depends on what kind of chemical label was used. But in general, the signal is weak.

Thus, there is a need in the mutation detection field for a rapid, low-cost, non-labor intensive, and clinically applicable technique that is able to detect any type of mutation occurring at a nucleic acid base at a specific predetermined position, and yet provides a strong and accurate detection signal.

### SUMMARY OF THE INVENTION

The present invention has met the herein before described need.

Even though the present invention shares some of the advantageous features associated with general primer extension based methods, such as the simplicity in design for testing for a mutation at a particular site, the present invention provides a method that overcomes the drawbacks associated with primer extension based methods as described above. The invention has wide applicability for detecting and identifying all types of mutations. It is cost-effective, timesaving, and less labor intensive than conventional methods.

Some of the key advantages of the invention over the above described methods are: 1) capability of detecting all types of mutations in only one reaction tube without necessarily employing gel electrophoretic size separation methods; 2) high degree of detection sensitivity by way of strong signal emitted due to incorporation of multiple labeled-nucleotides into the primer extension strand; and 3) high degree of accuracy because two or three different types of nucleotide or nucleotide analogue markers can be inserted into the primer extension strand at same time. These advantageous features provide an opportunity to use the invention to routinely test for the presence of a genetic mutation in any clinic based on this simple inventive procedure. The invention is also easily adaptable to automation for screening a large number of samples.

The invention relates to a method for detecting any mutation occurring at a predetermined nucleotide (target base) in a known nucleic acid sequence in a single reaction. The inventive method uses a primer extension analysis to detect the mutation. Preferably, the primer is complementary to and sequence-specifically hybridizes with the nucleic acid of interest at the position immediately adjacent to the predetermined nucleotide base to form a duplex, so that the target base in the nucleic acid of interest is an unpaired base immediately downstream of the 3' end of the primer. The primer extension reaction reagent includes one type of unlabeled terminator nucleotide (or optionally, no corresponding nucleotide base) along with three types of labeled (or optionally, differentially labeled or unlabeled) non-terminator nucleotides, wherein the terminator nucleotide is complementary to the target base at the predetermined position of the nucleic acid of interest. The labeled non-terminator nucleotides are not complementary to the target base. The incorporation of the terminator nucleotide into the 3' end of the primer complementary to the target base in the nucleic acid of interest will terminate the primer extension reaction without further incorporation of any labeled non-terminator nucleotides. If the target base was changed due to a mutation, a labeled non-terminator sequence-dependently incorporates into the primer. Thus, any labeled signal detected in the primer indicates that a mutation has occurred at the predetermined nucleic acid base site.

An object of the invention is to provide a method for detecting or quantifying a target nucleic acid in a sample comprising:
(a) preparing a primer complementary to a sequence immediately upstream of a target nucleotide base at a predetermined position in a template of a nucleic acid of interest;
(b) treating a sample containing the nucleic acid of interest, if the nucleic acid is double-stranded, so as to obtain unpaired nucleotide bases spanning the specific position, or directly employing step (c) if the nucleic acid of interest is single-stranded;
(c) annealing the primer from (a) with the target nucleic acid from (b) under high stringency conditions to obtain a primer-nucleic acid duplex, wherein the target nucleotide base in the nucleic acid of interest is the first unpaired base immediately downstream of the 3' end of the primer;
(d) mixing the primer-nucleic acid duplex from (c) with a primer extension reaction reagent comprising: (i) one type of terminator nucleotide or optionally, absence of a nucleotide, that is complementary to the target base at the predetermined position of the nucleic acid of interest, and (ii) three types of non-terminator nucleotides that are different from the terminator nucleotide in (i), and at least one type is optionally labeled with a detectable marker;
(e) performing the primer extension reaction by enzymatic or chemical means, wherein the incorporation of said terminator nucleotide or non-terminator nucleotide to the primer depends upon the identity of the unpaired nucleotide base in the nucleic acid template immediately downstream of the 3' end of the primer, and wherein incorporation of said terminator nucleotide in the sequence complementary to said target nucleotide base in the nucleic acid of interest will terminate said primer extension without incorporating any labeled non-terminator nucleotide into the primer, wherein said primer is not labeled, and further wherein, when the target nucleotide base is changed to any other type of nucleotide, one of the non-terminator nucleotides labeled with said detectable maker, or optionally not labeled with any marker if mass spectrometry is used as a detecting method, that is complementary to the mutated nucleotide base, is sequence-dependently incorporated into the primer by said primer extension reaction; and
(f) determining the presence and identity of the nucleotide base at the predetermined position in the nucleic acid of interest by detecting the incorporated labeled non-terminator in the primer.

In a preferred embodiment, in step (b), the nucleic acid base of interest is immediately adjacent to the nucleotide base to be identified at the predetermined position, and the nucleotide base to be identified is an unpaired base at a predetermined position immediately downstream of the 3' end of the duplex. In a preferred embodiment, in the method in step (d), the duplex from step (c) is contacted with at least one labeled non-terminator, and at least one unlabeled terminator. Alternatively, in step (d), the duplex from step (c) is contacted with non-terminators, wherein each non-terminator is labeled with same or different detectable label.

In another preferred embodiment, the method above can be practiced, wherein the template-dependent enzyme is *E. coli* DNA polymerase I or the "Klenow fragment" thereof, T4 DNA polymerase, T7 DNA polymerase *T. aquaticus* DNA polymerase, a retroviral reverse transcriptase, or combinations thereof.

In other preferred embodiments, the nucleic acid of the invention is a deoxyribonucleic acid, a ribonucleic acid, or a copolymer of deoxyribonucleic acid and ribonucleic acid. The primer is an oligodeoxyribonucleotide, an oligoribonucleotide, or a copolymer of deoxyribonucleic acid and ribonucleic acid. The template is a deoxyribonucleic acid, the primer is an oligodeoxyribonucleotide, oligoribonucleotide, or a copolymer of deoxyribonucleotides and ribonucleotides, and the template-dependent enzyme is a DNA polymerase. The template is preferably a ribonucleic acid, the primer is an oligodeoxyribonucleotide, oligoribonucleotide, or a copolymer of deoxyribonucleotides and ribonucleotides, and the template-dependent enzyme is a reverse transcriptase. Preferably, the template is a deoxyribonucleic acid, the primer is an oligoribonucleotide, and the enzyme is an RNA polymerase. Preferably, the template is a ribonucleic acid, the primer is an oligoribonucleotide, and the template-dependent enzyme is an RNA replicase.

In the above method, in step (d), the duplex from step (c) is contacted with at least one labeled non-terminator, and at least one terminator that is labeled differently from the non-terminator. In addition, in step (e), the label signal of the incorporated labeled non-terminator and at least one terminator that is labeled differently from the non-terminator are detected.

According to the method of the invention, the nucleic acid of interest has been synthesized enzymatically *in vivo,* synthesized enzymatically *in vitro,* or synthesized non-enzymatically. In another embodiment of the method of the invention, the oligonucleotide primer has been synthesized enzymatically *in vivo,* synthesized enzymatically *in vitro,* or synthesized non-enzymatically. In addition, the oligonucleotide primer can comprise one or more moieties that permit affinity separation of the primer from the unincorporated reagent and/or the nucleic acid of interest.

In particular, as a preferred embodiment, the oligonucleotide primer comprises biotin which permits affinity separation of the primer from the unincorporated reagent and/or nucleic acid of interest via binding of the biotin to streptavidin which is attached to a solid support. In another embodiment of the invention, the sequence of the oligonucleotide primer comprises a DNA sequence that permits affinity separation of the primer from the unincorporated reagent and/or the nucleic acid of interest via base pairing to a complementary sequence present in a nucleic acid attached to a solid support. In yet another embodiment of the invention, the nucleic acid of interest comprises one or more moieties that permit affinity separation of the nucleic acid of interest from the unincorporated reagent and/or the primer. The nucleic acid of interest can comprise biotin which permits affinity separation of the nucleic acid of interest from the unincorporated reagent and/or the primer via binding of the biotin to streptavidin which is attached to a solid support.

In a method of the invention, the sequence of the nucleic acid of interest comprises a DNA sequence that permits affinity separation of the nucleic acid of interest from the unincorporated reagent and/or the primer via base pairing to a complementary sequence present in a nucleic acid attached to a solid support. The oligonucleotide primer can be labeled with a detectable marker. The oligonucleotide primer can be labeled with a detectable marker that is different from any detectable marker present in the reagent or attached to the nucleic acid of interest. The nucleic acid of interest can be labeled with a detectable marker. The nucleic acid of interest is preferably labeled with a detectable marker that is different from any detectable marker present in the reagent or attached to the primer.

In another embodiment of the invention, the nucleic acid of interest comprises non-natural nucleotide analogs. The non-natural nucleotide analogs comprise deoxyinosine or 7-deaza-2'-deoxyguanosine. The nucleic acid of interest can be synthesized by the polymerase chain reaction.

In another method of the invention, the sample comprises genomic DNA from an organism, RNA transcripts thereof, or cDNA prepared from RNA transcripts thereof. The sample can comprise extragenomic DNA from an organism, RNA transcripts thereof, or cDNA prepared from RNA transcripts thereof. In the method of the invention the primer can be preferably separated from the nucleic acid of interest after the primer extension reaction in step (d) above by using appropriate denaturing conditions. Preferably, the denaturing conditions comprise heat, alkali, formamide, urea, glyoxal, enzymes, and combinations thereof. Even more preferably, the denaturing conditions comprise treatment with 0.2N NaOH.

The method of the invention can be practiced using nucleic acid from any organism, including plant, microorganism, virus, or bird. The organism can be a vertebrate or invertebrate. The organism is preferably a mammal. Even more preferably, the mammal is a human being. The mammal can be also a horse, dog, cow, cat, pig, or sheep.

These and other objects of the invention will be more fully understood from the following description of the invention, the referenced drawings attached hereto and the claims appended hereto.

### BRIEF DESCRIPTION OF THE DRAWINGS

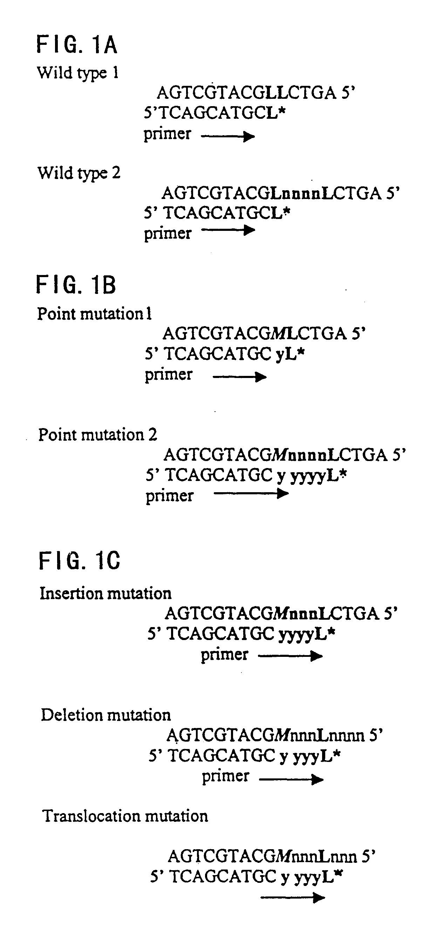

FIGS. 1A-1C. A schematic drawing of a preferred embodiment of the mutation detection method of the invention is shown. "L" represents the wild-type nucleotide, which can include A, G, C, T, or U. "L*" represents an unlabeled terminator such as a dideoxy nucleotide that is complementary to L. "M" represents a mutation at site L, and the mutant nucleotide can include A, G, C, T, or U. "W" represents a complementary nucleotide to M, and can include A, G, C, T, or U labeled with a detectable marker. "n" represents one or multiple nucleotides or nucleotide analogues, including A, G, C, T, and U. "y" represents a nucleotide or nucleotide analogue, including A, G, C, T, or U, labeled with a detectable marker and complementary to M or n.

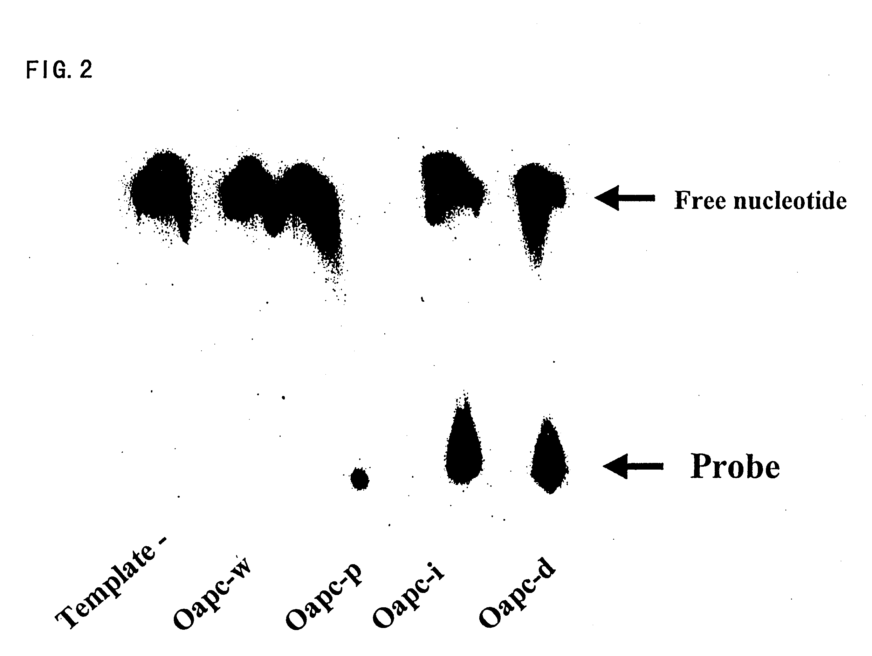

FIG. 2.1 µl of the STA reaction mixture was applied to a thin layer chromatography strip and then the strip was subjected to solvent containing 1 M NaCl and 1 M HCl. The strip was then dried at room temperature for 10 min and exposed to Kodak film for 30 minutes, and the film was developed by auto-film developer. The templates used STA test are marked under each strip. The top arrow indicates the free nucleotide front and the arrow at the bottom indicates the primer extended strand incorporated with [-³²P] dCTP.

### DETAILED DESCRIPTION OF THE INVENTION

As used herein, "nucleic acid" or "nucleotide" can be a deoxyribonucleic acid, a ribonucleic acid, or a copolymer of deoxyribonucleic acid and ribonucleic acid. The sample of nucleic acids can be natural or synthetic. The sample of nucleic acid can be naturally occurring nucleic acid, and can be obtained from any organism. Some examples of organisms to which the method of the present invention is applicable include plants, microorganisms, viruses, birds, vertebrates, invertebrates, mammals, human beings, horses, dogs, cows, cats, pigs, or sheep.The target nucleic acid can occur naturally, or can be synthesized enzymatically *in vivo,* synthesized enzymatically *in vitro,* or synthesized non-enzymatically.

The sample containing the nucleic acid or acids of interest can comprise genomic DNA from an organism, RNA transcripts thereof, or cDNA prepared from RNA transcripts thereof. The sample containing the nucleic acid or acids of interest can also comprise extragenomic DNA from an organism, RNA transcripts thereof, or cDNA prepared from RNA transcripts thereof. Also, the nucleic acid or acids of interest can be synthesized by the polymerase chain reaction.

The nucleic acid of interest can comprise non-natural nucleotide analogs such as deoxyinosine or 7-deaza-2-deoxyguanosine. These analogues destabilize DNA duplexes and could allow a primer annealing and extension reaction to occur in a double-stranded sample without completely separating the strands.

The nucleic acid of interest can comprise one or more moieties that permit affinity separation of the nucleic acid of interest from the unincorporated reagent and/or the primer. For example, the nucleic acid of interest can comprise biotin which permits affinity separation of the nucleic acid of interest from the unincorporated reagent and/or the primer via binding of the biotin to avidin and its analogue which is attached to a solid support. The sequence of the nucleic acid of interest can comprise a DNA sequence that permits affinity separation of the nucleic acid of interest from the unincorporated reagent and/or the primer via base pairing to a complementary sequence present in a nucleic acid attached to a solid support. The nucleic acid of interest can be labeled with a detectable marker; this detectable marker can be different from any detectable marker present in the reagent or attached to the primer.

In this regard, the term "normal nucleotide" or "normal base" is defined as the wild-type or previously known standard nucleotide base from which a mutation is sought to be identified at the base site. By "standard nucleotide base", it includes any known base, which may include wild-type or a known mutant base so long as the base is known and it is desired to know its variant. Thus, as an example, normal base can be a known wild-type base for which a mutation is sought at the position. Inversely, the known base can be a known mutant for which the presence of a wild-type base is sought at the position. Alternatively, the known normal base can be a known mutant for which another mutant variant base is sought. Therefore, the method of the invention can be applied to any known sequence that can be used to determine the presence of any other base variant at the site.

As used herein, the term "primer" or "oligonucleotide primer" refers to an oligonucleotide which is capable of acting as a point of initiation of synthesis when placed under conditions that allow for synthesis of a primer extension product which is complementary to a nucleic acid (template) strand, in the presence of various factors such as for example, nucleotides and enzymes such as DNA polymerase, and at a suitable temperature and pH.

The term "primer" is alternatively defined as any nucleic acid fragment obtained from any source. For example, the primer can be produced by fragmenting larger nucleic acid fragments such as genomic DNA, cDNA or DNA that has been obtained through PCR. In other words, the nature of the primer is not limited by how the primer is obtained, whether it be by fragmenting naturally or synthetically occurring nucleic acid or by synthesizing the nucleic acid primer. Furthermore, the primer can be oligodeoxyribonucleotide, a copolymer of oligodeoxyribonucleotides, an oligoribonucleotides, a copolymer of ribonucleotides, or a copolymer of deoxyribonucleotides and ribonucleotides. The primer can be either natural or synthetic. The oligonucleotide primer can be synthesized either enzymatically *in vivo,* enzymatically *in vitro,* or non-enzymatically *in vitro.* The primer can be labeled with a detectable marker; this detectable marker can be different from any detectable marker present in the reagent or attached to the nucleic acid of interest. In addition, the primer must possess sequence corresponding to the flanking sequence at a specific position of interest adjacent to, and upstream of, the nucleotide base to be identified.

In addition, the primer must be capable of hybridizing or annealing with nucleotides present in the nucleic acid of interest. One way to accomplish the desired hybridization is to have the template-dependent primer be substantially complementary or fully complementary to the known base sequence.

The oligonucleotide primer can comprise one or more moieties that permit affinity separation of the primer from the unincorporated reagent and/or the nucleic acid of interest. Such affinity moeties include, but are not limited to, digitonin, magnetic beads, and ligands, such as protein ligands, including antibodies. Preferably, the moiety is biotin. In the case of using biotin, the primer comprising biotin permits affinity separation of the primer from the unincorporated reagent and/or nucleic acid of interest via binding of the biotin to avidin and its analogue which is attached to a solid support. The sequence of the oligonucleotide primer can comprise a DNA sequence that permits affinity separation of the primer from the unincorporated reagent and/or the nucleic acid of interest via base pairing to a complementary sequence present in a nucleic acid attached to a solid support.

As used herein, the term "primer extension reaction" refers to the reaction conditions in which the template-dependent nucleic acid synthesis reaction is carried out. The conditions for the occurrence of the template-dependent, primer extension reaction can be created, in part, by the presence of a suitable template-dependent enzyme. Some of the suitable template-dependent enzymes are DNA polymerases. The DNA polymerase can be of several types. The DNA polymerase must, however, be primer and template dependent. For example, E. *coli* DNA polymerase I or the "Klenow fragment" thereof, T4 DNA polymerase, T7 DNA polymerase ("Sequenase"), T. *aquaticus* DNA polymerase, or a retroviral reverse transcriptase can be used. RNA polymerases such as T3 or T7 RNA polymerase could also be used in some protocols. Depending upon the polymerase, different conditions must be used, and different temperature ranges may be required for the hybridization and extension reactions.

As used herein, the term "primer extension strand" includes the strand that is formed opposite the template in a duplex after the primer has been added. Preferably, the extension of the primer has terminated by the binding of the terminator to the template.

As used herein, the term "template" is defined as a nucleic acid, including double strand DNA, single strand DNA and RNA, or any modification thereof, and can be any length or sequence.

As used herein, the term "terminator" or "chain terminator" is meant to refer to a nucleic acid base, such as A, G, C, T or U, or an analogue that effectively terminates the primer extension reaction when it is incorporated into the primer extension strand opposite the template strand. Preferably, the terminator is a dideoxynucleotide. Also preferably, the terminator is either unlabeled or is labeled so that it is distinguished from the label on the non-terminator. Also as used herein, when the term "terminator" or "chain terminator" are referred to in the singular, it does not mean that a single nucleotide molecule is used. Rather, the singular form of the term "terminator" refers to the type of nucleotide, nucleic acid base or nucleic acid analogue that is used in the assay. For example, if the terminator is ddA, then all of the ddA's in the aggregate are referred to in the singular form, and not just a single molecule of ddA. Alternatively, the "terminator" may be the absence of the specific type of nucleotide so that primer extension is stopped by the lack of the specific nucleotide at the locus. For example, if it is desired that the primer extension reaction be stopped opposite a "C" on the template strand, the non-terminating bases A, T and G should be included in the primer extension reaction mixture, but not "G", which is the complement of "C". Thus, the absence of the complementary base will cause termination of the primer extension reaction with a similar result as adding a dideoxy terminator nucleotide, for example.

As used herein, the term "non-terminator" or "non-chain terminator" includes a nucleotide base that does not terminate the extension reaction when it is incorporated into the primer extension strand. Preferably, at least one non-terminator in the primer extension reaction is labeled. Also as used herein, when the term "non-terminator" or "non-chain terminator"are referred to in the singular, it does not mean that a single nucleotide molecule is used. Rather, the singular form of the term "non-terminator" refers to the type of nucleotide, nucleic acid base or nucleic acid analogue that is used in the assay. For example, if the terminator is G, then all of the G's in the aggregate are referred to in the singular form, and not just a single molecule of G.

As used herein, the term "mutant" or "mutation" indicates any base on the template strand that is different from the wild-type or normal base. The mutation that can be detected using the method of the instant invention can be any type of mutation at all, including, single base mutation, insertion, deletion, or gene translocation, so long as the base on the template directly opposite to the base immediately 3' to the annealed primer is affected.

As used herein, the term "label" refers to any molecule that is linked to the terminator or non-terminator nucleotide to provide a detectable signal. The label may be radioactive, chemiluminescent, protein ligand such as an antibody, or if a fluorescent group is used, a different fluorescent group may be used for each type of non-terminating nucleotide base. These fluorescent tags would have the property of having spectroscopically distinguishable emission spectra.

Alternatively, the method of determining the level of incorporation of a nucleotide base in the primer extension product can be measured by mass spectrometry techniques as exemplified in U.S. Patent No. 5,885,775, which is incorporated herein by reference in its entirety.

As used herein, the phrase "high stringency hybridization conditions" refers to nucleic hybridization conditions, such as but not limited to a wash condition of 0.1XSSC, at 42°C. Hybridization conditions generally can be found in general Molecular Biology protocol books, such as Ausubel et al., *Current Protocols in Molecular Biology* Greene and Wiley, pub. (1994), which is incorporated herein by reference in its entirety.

As used herein, "thin layer chromatography (TLC)" can be carried out in paper medium based on cellulose products, but can be made of any substance that allows for molecules to be finely divided and formed into a uniform layer. This substance includes, but is not limited to, inorganic substances such as silica gel, aluminum oxide, diatomaceous earth or magnesium silicate. Organic substances include, but are not limited to, cellulose, polyamide, or polyethylene powder. Thin layer chromatography methods are described generally in Chemical protocol books, such as generally set forth in Freifelder, *Physical Biochemistry - Applications to Biochemistry and Molecular Biology, second ed.,* published by Freeman and Co. (1982), which is incorporated herein by reference in its entirety, especially Chapter 8, which discusses chromatographic techniques, and in particular thin layer chromatography at pages 229-232.

It can be appreciated by a person of skill in the art that the terminator can be labeled with a different label from the non-terminator, which can then be used to differentiate between incorporation of terminator or non-terminator in the primer extension strand. The terminator exemplified as being the absence of the particular type of nucleotide in the present application only for purposes of simplicity of illustration, but this illustration should not be construed to limit the claims in any way. Differentially labeled or unlabeled terminator is also encompassed by the invention, so long as the label on the terminator is different from the label on the non-terminator.

It can also be appreciated by a person of skill in the art that so long as the sequence of the template is at least partially known, a primer can be designed that binds to the template strand such that the binding of the primer on the template strand can occur. It can also be appreciated by a person of skill in the art that the method of the invention can be practiced by using several primers in one or more assay tube.

A feature of the method of the invention is that strong signal can be generated if the non-terminators are uniformly labeled because of the additive signal effect achieved by the incorporation of several labeled non-terminators incorporated in the primer extension strand when there is a mutation at the predetermined site. This generates an advantageous signal strength over conventional mutation detection methods that incorporate only a single label per each primer extension strand. Accuracy is enhanced when signals are observed from using different labels specific to various terminators or non-terminators.

The following examples are offered by way of illustration of the present invention, and not by way of limitation.

### EXAMPLES

### Example 1

Sequence of human APC gene was selected as a target sequence for the STA test of the invention. Oligonucleotides corresponding to the wild-type APC sequence 4317-4347 and three different types of mutations were synthesized and used as templates. The primers employed in the STA test are listed in Table 1.

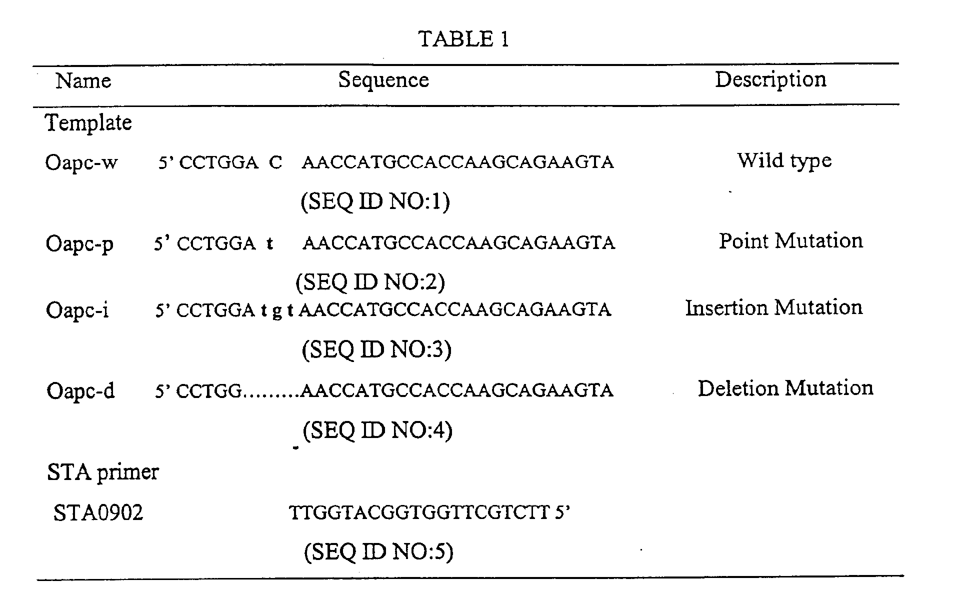

STA: Each STA reaction was performed in 20 µl buffer (10 mM Tris-HCl, pH7.5, 50 mM KCl, and 5 mM MgCl₂) containing 50ng of template oligonucleotide, 1 µM primer, 2 units of DNA Polymerase, 1 µl of [α-³²P]-labeled dCTP (250 µCi/ml, 3000 Ci/mmol Dupont-New England Nuclear), dATP, dTTP, and 1µl of non-labeled dd GTP. The mixture was incubated at 37 °C for 30 minutes and heated at 100 °C for 3 minute. 1 µl of the reaction mixture was applied to a TI strip, which is a strip of thin layer chromatography (TRIM USA, MD). The strips were extended for 10 minutes with solution containing 1M HCl and 1M NaCl. The primers were completely separated from unincorporated nucleotide on the TI strip by this procedure. The labeled primer was .visualized by autoradiography and the radioactivity was counted by scintillation counter (Beckman LS 5000). The autoradiogram is shown in Fig. 2, and then counting the results for the corresponding autoradiogram is shown in Table 2.

**TABLE 2**

| Template | Total Counts | Labeled Primer |
|---|---|---|
| No template | 76,675 | 95 |
| Oapc-w | 79,599 | 117 |
| Oapc-p | 82,584 | 4,821 |
| Oapc-i | 75,376 | 8,602 |
| Oapc-d | 100,634 | 6,571 |

The counts shown in Table 2, right column represents the amount of [α-³²P]-dCTP incorporated into the primer extension Oapc-w, a wild type oligonucleotide, is serves as the template. The first unpaired nucleotide after primer annealing is C, which is complementarily matched by the terminator ddG. When the reaction of template-dependent primer extension was started, the terminator ddG was soon incorporated into 3' end of the primer as a first extended nucleotide and the further incorporation of labeled nucleotides was blocked by the bound ddG. As a result, the primer has been extended by only one nucleotide base, which is the terminator nucleotide. Since it is not possible for other nucleotide bases to bind to the template after the terminator has bound, the primer extension reaction has stopped. The radioactive count in the Oapc-w sample showed that the count was similar to the sample without any template, i.e., background control.

In comparision, Oapc-p is an oligonucleotide with a point mutation. The template was created by replacing the wild-type C to the mutant T at the first unpaired nucleotide at the 3' end of the wild-type template. In this case, the dATP instead of the terminator ddG was complementarily matched to the mutated nucleotide T, when the primer extension reaction was started. The primer extension reaction stopped after the terminator ddG was incorporated into the position opposite the C residue on the template strand.

In the mutant oligonucleotide encompassing an insertion mutation, Oapc-i in Tables 1 and 2, the first unpaired nucleotide is T, which is not complementary to the terminator ddG. In this case, the primer is extended by the binding of dATP opposite it on the primer extension strand, and then the primer is further elongated by the addition of [α-³²P]-dCTP, dATP, two [α-³²P]-dCTP's and dATP. Sequential incorporation of ddG by the nucleotide polymerase first encountering C terminates the elongation process. The final result is that three [α-³²P]-dCTPs were incorporated into the primer extension strand.

As in the insertion mutant, the oligonucleotide deletion mutant, Oapc-d (Tables 1 and 2), was assayed using the inventive STA reagent and method. The primer was extended by incorporating in order, two [α-³²P]-dCTP, dATP, and terminated with ddG. Thus, two [α-³²P]-CTP's were incorporated into the primer extension strand. These results provide strong evidence that the inventive STA method can detect all types of mutations. The STA method can identify the presence of any type of mutation by performing only one test.

Particularly in the deletion and insertion mutations (Oapc-I and Oapc-d) multiple labeled nucleotides were incorporated into the primer extension strand. This multiple labeling dramatically enhanced the detection sensitivity. In addition, the test sensitivity can be further increased by using different nucleotides labeled with the same detection marker. For example, all of the non-terminators can be labeled, such as, [α-³²P]-CTP, [α-³²P]-ATP, [α-³²P]-TTP, in extending the primer.

The multiple labeling also provides an opportunity to label the non-terminator nucleotides with different detectable markers to distinguish each non-terminator nucleotide base. For example, the nucleotides can be labeled with different fluorescent dyes, the primer is then extended carrying the different fluorescent labels. Detection of the different signals at the same time will increase the accuracy of the STA test. These advanced multiple labeling features associated with the inventive STA reagent and method provides greater mutant detection sensitivity and accuracy over methods known in the art.

### Example 2

PCR products of human APC gene were used as a test sample. A fragment of APC gene was PCR amplified by using standard PCR protocol. Human APC cDNA was used as a template. The primers used for PCR are listed in Table 3.

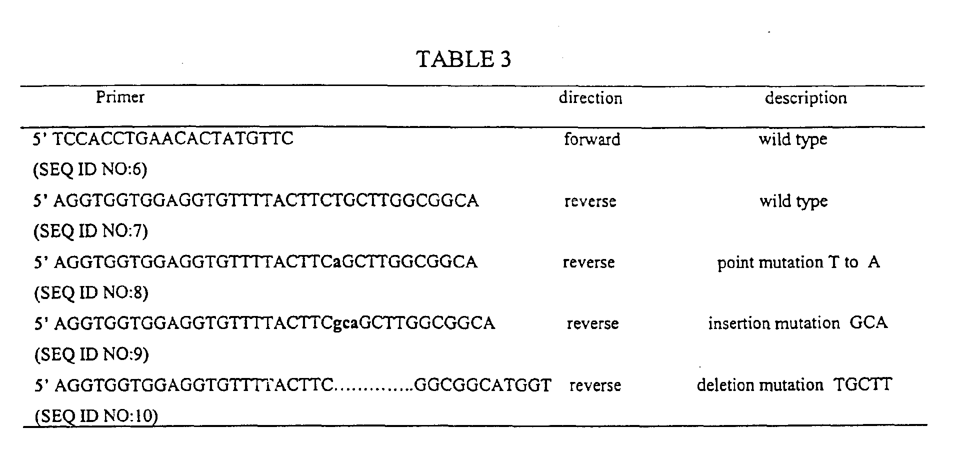

Four different PCR products size about 200 bp were generated by combination of the primers. They are APC-w: wild-type; APC-p: having a point mutation; APC-i: having an insertion mutation and APC-d: having a deletion mutation. The PCR products were applied to 1% agarose gel to remove the template and free nucleotides. The products were then purified by Qiax DNA purification Kit (Qiagen). The STA primer is designed as 5' -AGGTGGTGGAGGTGTTTTACTTC - 3' (SEQ ID NO: 11) and STA reactions were performed in a total volume of 20 µl in a buffer containing 10 mM Tris-HCl, pH 8.3, 50 mM KCl, 2 mM MgCl₂, 0.05 pmol double strand PCR product, 5 pmol primers, 20pM of dATP, dGTP, 1 µCi of [α-³²P]-labeled CTP, 20µM non-labeled dideoxy TTP and 2 units of Taq DNA Polymerase. Twenty cycles of 94 °C for 20 s, 55 °C for 1 min were performed in a thermocycler (Perkin Elmer, GeneAmp 9600). 1 pl of STA product was applied to Trim Strip, a thin layer chromatography strip made by TRIM Corporation in Japan, and radioactivity was counted as described in Example 1. The results are shown in Table 4.

**TABLE 4**

| | Total Counts | Labeled Primer |
|---|---|---|
| No template | 182,245 | 343 |
| Papc-*w* | 208,271 | 595 |
| Papc-*p* | 197,494 | 5,568 |
| Papc-*i* | 176,984 | 10,372 |
| Papc-*d* | 195,570 | 12,010 |

The primer in all three types of mutant samples has been extended with [α-³²P] dCTP. The intensity of the strength of the signal generated by the primer extension incorpoating the label correlates well with the number of labeled nucleotides that are carried in the primer extension strand.

### Example 3

The inventive STA reagent and method was applied to RNA fragments of human APC gene. The PCR products of human APC gene in Example 2 were ligated into TA cloning Vector 3.1 (TA cloning kit, Invitrogen). Four vectors were constructed and they are listed in Table 5.

**TABLE 5**

| Vector | Insert | description | Name of RNA products |
|---|---|---|---|
| Tapc-w | APC-w | Wild type | Rapc-w |
| Tapc-p | APC-p | Point Mutation | Rapc-p |
| Tapc-I | APC-i | Insertion Mutation | Rapc-I |
| Tapc-d | APC-d | Deletion Mutation | Rapc-d |

RNA species corresponding to each vector was synthesized using an *in vitro* RNA synthesis kit (Promega, WI). The RNA was synthesized at 37 °C for 1 hour in buffer containing 2µg of vector and T7 Polymerase. The reaction was stopped by adding LiCl and 100% ethanol. After incubation at -20 °C for 15 min., the RNA was precipitated by spinning in a centrifuge at 14,000g for 15 min., and the purified RNA was resuspended in RNase-free water. 5µg RNA was mixed with the STA primer described in Example 2 in a total volume of 10 µl buffer containing 10 mM Tris-HCl pH 7.6, 50 mM NaCl, and 10 mM KCI. The mixture was heat denatured at 65 °C for 3 minutes followed by quenching on ice for 2 minutes. STA reaction was performed as described in Example 1, the buffer containing 1 µl of [α-³²P]-labeled dCTP (250 µCi/ml, 3000 Ci/mmol Dupont-New England Nuclear), 10 µM dATP, dGTP, and 10 µM of non-labeled ddTTP, and 20 units of reverse transcriptase. After incubating at 40 °C for 15 minutes, the reaction was stopped by heating at 100 °C for 2 minutes. 1 µl reaction product was applied to Trim Strip and the radioactivity was counted as described in Example 1. The results are shown in Table 6.

**TABLE 6**

| sample | Total Counts | Labeled Primer |
|---|---|---|
| No template | 167,496 | 690 |
| Rapc-w | 172,734 | 435 |
| Rapc-p | 166,979 | 1,745 |
| Rapc-i | 170,801 | 7,348 |
| Rapc-d | 174,888 | 7,360 |

All of the above steps involve chemistries, manipulations, and protocols that have been, or are amenable to being, automated. Thereby, incorporation of the preferred mode of practice of this invention into the operation of a suitably programmed robotic workstation should result in significant cost savings and increases in productivity for virtually any diagnostic procedure that depends on the detection of specific nucleotide sequences or sequence differences in nucleic acids derived from biological samples.

All of the references cited herein are incorporated by reference in their entirety.

## Claims

1. A method for detecting or quantifying a target nucleic acid in a sample comprising:
(a) preparing a primer complementary to a sequence immediately upstream of a target nucleotide base at a predetermined position in a template of a nucleic acid of interest;
(b) treating a sample containing the nucleic acid of interest, if the nucleic acid is double-stranded, so as to obtain unpaired nucleotide bases spanning the specific position, or directly employing step (c) if the nucleic acid of interest is single-stranded;
(c) annealing the primer from (a) with the target nucleic acid from (b) under high stringency conditions to obtain a primer-nucleic acid duplex, wherein the target nucleotide base in the nucleic acid of interest is the first unpaired base immediately downstream of the 3' end of the primer;
(d) mixing the primer-nucleic acid duplex from (c) with a primer extension reaction reagent comprising: (i) one type of terminator nucleotide or optionally, absence of a nucleotide, that is complementary to the target base at the predetermined position of the nucleic acid of interest, and (ii) three types of non-terminator nucleotides that are different from the terminator nucleotide in (i), and at least one type is optionally labeled with a detectable marker;
(e) performing the primer extension reaction by enzymatic or chemical means, wherein the incorporation of said terminator nucleotide or non-terminator nucleotide to the primer depends upon the identity of the unpaired nucleotide base in the nucleic acid template immediately downstream of the 3' end of the primer, and wherein incorporation of said terminator nucleotide in the sequence complementary to said target nucleotide base in the nucleic acid of interest will terminate said primer extension without incorporating any labeled non-terminator nucleotide into the primer, wherein said primer is not labeled, and further wherein, when the target nucleotide base is changed to any other type of nucleotide, one of the non-terminator nucleotides labeled with said detectable maker, or optionally not labeled with any marker if mass spectrometry is used as a detecting method, that is complementary to the mutated nucleotide base, is sequence-dependently incorporated into the primer by said primer extension reaction; and
(f) determining the presence and identity of the nucleotide base at the predetermined position in the nucleic acid of interest by detecting the incorporated labeled non-terminator in the primer.

2. The method according to claim 1, wherein the primer is a fragment of deoxyribonucleic or ribonucleic acid, an oligodeoxyribonucleotide, an oligoribonucleotide, or a copolymer of deoxyribonucleic acid and ribonucleic acid.

3. The method according to claim 1, wherein the nucleic acid of interest is deoxyribonucleic acid, a ribonucleic acid, or a copolymer of deoxyribonucleic acid and ribonucleic acid.

4. The method according to claim 1, wherein the target nucleotide is defined as any known base, which include wild-type or a known mutant base so long as the base is known and it is desired to know its variant.

5. The method according to claim 1, wherein the terminator nucleotide is a dideoxyribonucleotide and the non-terminator nucleotide is a deoxyribonucleotide or a ribonucleotide.

6. The method according to claim 1, wherein the terminator nucleotide is unlabeled.

7. The method according to claim 1, wherein the terminator nucleotide is labeled with a detectable marker that is different from the marker on the non-terminators.

8. The method according to claim 1, wherein in step (d), the duplex from step (c) is contacted with non-terminator nucleotides, wherein each non-terminators is labeled with the same or different detectable marker.

9. The method according to claim 1, wherein said detectable marker comprises an enzyme, radioactive isotope, a fluorescent molecule, or a protein ligand.

10. The method according to claim 1, wherein said detecting is carried out by mass spectrometry.

11. The method according to claim 1, wherein said enzyme is template-dependent.

12. The method of claim 11, wherein the template-dependent enzyme is DNA polymerase.

13. The method according to claim 12, wherein the DNA polymerase is *E. coli* DNA polymerase I or the "Klenow fragment" thereof, T4 DNA polymerase, T7 DNA polymerase, or *T. aquaticus* DNA polymerase.

14. The method according to claim 11, wherein said enzyme is RNA polymerase or reverse transcriptase.

15. The method according to claim 1, wherein the primer comprises one or more moieties that permit affinity separation of the primer from the unincorporated reagent and/or the nucleic acid of interest.

16. The method according to claim 1, wherein the primer comprises one or more moieties that allows linking the primer to a solid surface.

17. The method according to claim 15, wherein the moieties comprises biotin or digitonin.

18. The method according to claim 16, wherein the moieties comprises biotin or digitonin.

19. The method according to claim 15, wherein the moieties comprises a DNA or RNA sequence that permits affinity separation of the primer from the unincorporated reagent and/or the nucleic acid of interest via base pairing to a complementary sequence present in a nucleic acid attached to a solid support.

20. The method according to claim 16, wherein the moieties comprises a DNA or RNA sequence that permits affinity separation of the primer from the unincorporated reagent and/or the nucleic acid of interest via base pairing to a complementary sequence present in a nucleic acid attached to a solid support.

21. The method according to claim 15, wherein the moieties comprises a DNA or RNA sequence that allows the primer to link to a solid support via base pairing to a complementary sequence present in solid surface.

22. The method according to claim 16, wherein the moieties comprises a DNA or RNA sequence that allows the primer to link to a solid support via base pairing to a complementary sequence present in solid surface.

23. The method according to claim 1, wherein the nucleic acid of interest has been synthesized enzymatically *in vivo, in vitro,* or synthesized non-enzymatically.

24. The method according to claim 1, wherein the nucleic acid of interest is synthesized by polymerase chain reaction.

25. The method according to claim 1, wherein the nucleic acid of interest comprises non-natural nucleotide analogs.

26. The method according to claim 25, wherein the non-natural nucleotide analogs comprise deoxyinosine or 7-deaza-2'-deoxyguanosine.

27. The method according to claim 1, wherein the sample comprises genomic DNA from an organism, RNA transcripts thereof, or cDNA prepared from RNA transcripts thereof.

28. The method according to claim 1, wherein the sample comprises extragenomic DNA from an organism, RNA transcripts thereof, or cDNA prepared from RNA transcripts thereof.

29. The method according tc claim 27, wherein the organism is a plant, microorganism, bacteria, virus.

30. The method according to claim 28, wherein the organism is a plant, microorganism, bacteria, virus.

31. The method according to claim 27, wherein the organism is a vertebrate or invertebrate.

32. The method according to claim 28, wherein the organism is a vertebrate or invertebrate.

33. The method according to claim 27, wherein the organism is a mammal.

34. The method according to claim 28, wherein the organism is a mammal.

35. The method according to claim 27, wherein the organism is a human being.

36. The method according to claim 27, wherein the organism is a human being.
